(19) Europäisches Patentamt
European Patent Office
Office européen des brevets

(11) Publication number: 0 220 966
A2

(12) EUROPEAN PATENT APPLICATION

(21) Application number: 86308484.4

(22) Date of filing: 30.10.86

(51) Int. Cl.$^4$: C 07 K 3/28
C 12 P 21/02, C 07 K 13/00,
A 61 K 37/02

(30) Priority: 30.10.85 US 792815 22.05.86 US 866213

(43) Date of publication of application:
06.05.87 Bulletin 87/19

(84) Designated Contracting States:
AT BE CH DE ES FR GB IT LI LU NL SE

(71) Applicant: CETUS CORPORATION
1400 Fifty-Third Street
Emeryville California 94608 (US)

(72) Inventor: Lin, Leo Shun-Lee
1317 Chesterton Way
Walnut Creek California 94596 (US)

Dorin, Glenn
155 Humbolt
San Rafael California 94901 (US)

Yamamoto, Ralph
42 Emerson Street
San Francisco California 94118 (US)

Hanisch, Wolfgang Helmut
23 Barton Parade
Balmoral Heights Brisbane Queensland (US)

Thomson, James William
811 Talbot Avenue
Albany California 94706 (US)

Wolfe, Sidney Norman
2335 Alva Street
El Cerrito California 94530 (US)

(74) Representative: Bizley, Richard Edward et al
BOULT, WADE & TENNANT 27 Furnival Street
London EC4A 1PQ (GB)

(54) **Purification method for proteins.**

(57) Tumor necrosis factor (TNF) may be purified using a hydrophobic support in a chromatographic column that is then developed, preferably using high pressure liquid chromatography. Also, recombinantly produced biologically active proteins may be purified by passing a solution containing the protein through a continuous porous hydrophobic membrane, and recovering the fraction enriched in the desired protein. TNF and recombinant ricin toxin A chain may be purified. A highly purified TNF comprising at least 95% TNF, as determined by SDS-PAGE analysis, with an endotoxin content of less than 0.1 nm/mg TNF that is substantially free of pyrogens by the USP rabbit pyrogen test at a dosage range of 1.0 to 2.4 x $10^5$ U/Kg is obtained.

EP 0 220 966 A2

**Description**

PURIFICATION METHOD FOR PROTEINS

This invention relates to a process for the purification of biologically active proteins. In particular it relates to a process for the purification and recovery of polypeptides having the biological activity of tumor necrosis factor (TNF). The process herein involves purification of TNF from the cellular extract or the medium of recombinant host cells or from native sources. The process disclosed herein is especially useful in the recovery of substantially pure homogeneous biologically active recombinant TNF which is free of pyrogenic activity other than that which may be caused by TNF itself. The invention also relates to the substantially pure homogeneous biologically active TNF which is free of pyrogenic activity other than that which may be caused by TNF itself. The invention furthermore relates to a process for the purification of biologically active proteins that are hydrophobic at physiological pH, such proteins being in this regard like TNF.

Processes for the purification of proteins are generally known and include such techniques as ion exchange chromatography, adsorption chromatography, gel electrophoresis, ammonium sulfate precipitation, and gel filtration.

Although each of these techniques is known, it is impossible to predict the extent to which any of the above-listed techniques is applicable to the purification of a given protein. Various factors including the extent of purification desired. the extent of acceptable loss of biological activity of the protein, and degree of homogeneity of the protein desired, require extensive experimentation to optimize the purification of the products.

Human TNF has been purified as a native protein from culture supernatants of induced HL-60 cells by a combination of anion exchange chromatography and reverse phase high pressure liquid chromatography (HPLC), with elution in a linear gradient of acetonitrile (Wang, A. M., et al., Science (1985) 228:149-154). Similar procedures had been previously employed (Matthews, N., Br. J. Cancer (1981) 44:418) without resulting in a homogeneous preparation. However, this technique is not optimally efficient even for the native TNF secreted from, for example, HL-60 or other TNF secreting cell lines, and is inappropriate for recombinantly produced TNF, due to substantial inactivation of TNF biological activity at low pH.

European Patent Publication No. 168,214 published January 15, 1986 discloses a process for purifying TNF by the steps of obtaining a TNF solution from cell culture supernatants or lysates, removing solids, adsorbing TNF from the remaining supernatant onto a silicate support, eluting TNF from the silicate support, chromatographing TNF on a tertiary amino anion exchange resin, and chromatographing TNF on an anion exchange resin containing quaternary ammonium substituents. Optional purification steps including chromatofocusing to concentrate and purify the product or passage through a sieving gel such as Sephadex G-25 are disclosed. As a hydrophobic support, EP Publication 168,214 discloses the use of silicate, polyolefin and alkyl Sepharose. The TNF is eluted from the silicate using a polyol, preferably ethylene glycol in a 10-30% range, with a 20% (v/v) concentration preferred. Further purification, according to the process, requires adsorption onto a tertiary or quaternary amino anion exchange resin such as DEAE cellulose, QAE Sephadex or the product sold under the tradename Mono Q. Purification to homogeneity, according to the process, is accomplished only upon further separation on sodium dodecyl sulfate-polyacrylamide gel electrophoresis (SDS-PAGE) or C4 reverse phase HPLC. These latter steps are accompanied, however, by substantial loss of biological activity.

The present invention provides methods for purifying TNF that produce a substantially homogeneous TNF without recourse to reverse phase HPLC or SDS-PAGE electrophoresis. In one aspect, the invention substitutes a hydrophobic chromatographic support for the reverse phase HPLC of Wang et al., supra and Matthews et al., supra and permits isolation of pure TNF and various TNF muteins using a decreasing salt concentration gradient. In another aspect, a filtration step through a hydrophobic porous matrix is used that offers substantial recovery of active recombinant TNF proteins and substantially complete removal of host cell proteins. endotoxins and pyrogens when the host cell is a Gram-negative microorganism, before the use of any chromatographic techniques in purifying the TNF. As a result, large scale recovery of the material is possible.

In one aspect, the invention relates to a method for obtaining partially purified biologically active proteins, preferably those that are hydrophobic at physiological pH, wherein such proteins are produced in a recombinant host, comprising the step of passing a fluid containing the biologically active protein through a continuous hydrophobic porous matrix and recovering the partially purified biologically active protein.

In another aspect, the invention relates to a method for purifying tumor necrosis factor (TNF) comprising the steps of:

(a) treating an aqueous mixture containing TNF with a hydrophobic non-silica interaction matrix under a first salt concentration condition effective to result in retention of TNF on the hydrophobic matrix, but ineffective to precipitate the TNF, and

(b) eluting the TNF from the hydrophobic matrix chromatographically by decreasing the salt concentration below the first salt concentration of step (a).

Typically a buffer containing about 1.5-2 M ammonium sulfate or its equivalent in ionic strength, the TNF, and small quantities of other proteins are adsorbed to the hydrophobic matrix in step (a). Preferably, step (b) is carried out in a continuous gradient, although a stepwise gradient may also be employed. The preferred TNF

for this method is a recombinant form of TNF produced in bacterial hosts. The TNF thus produced may be substantially similar to the mature form or may contain deliberate or fortuitous amino acid modifications including N-terminal sequence deletions.

This procedure results in clean separation of the TNF from any contaminating proteins, thus producing homogeneous TNF on SDS-PAGE. This TNF can be conveniently assessed for side-chain modifications or other modifications using isoelectric focusing or other analyses that alter the isoelectric points.

In another aspect, a method is provided for partially purifying TNF from a TNF-containing fluid obtained from TNF-producing recombinant host cells characterized by the steps of: (a) passing the TNF-containing fluid through a continuous hydrophobic porous matrix, and (b) recovering the partially purified TNF therefrom.

In another aspect, the invention includes the partially purified TNF produced by the latter method. The partially purified TNF comprises at least about 20% by weight of the total TNF produced by the recombinant host cells, and about 40 to 50% by weight of the total recovered protein, and has an endotoxin content of 10 ng/ml-10 μg/ml.

In another aspect, the invention is a process for obtaining a purified TNF from a TNF-containing fluid obtained from TNF-producing recombinant host cells characterized by the steps of (a) passing the TNF-containing fluid through a continuous hydrophobic porous matrix to produce a partially purified TNF, (b) further purifying said partially purified TNF by at least one hydrophobic interaction matrix chromatography step and at least one anion exchange matrix chromatography step, and (c) recovering a purified TNF having a TNF content of at least 95% as determined by SDS-PAGE analysis and an endotoxin content of less than 0.1 ng/mg TNF.

In one embodiment of this process, the anion exchange chromatography step preceeds the hydrophobic interaction matrix chromatography step.

In another embodiment of this process, the hydrophobic interaction matrix chromatography step preceeds the anion exchange chromatographic step.

Other optional steps of the process according to the invention include size exclusion chromatography and concentration steps. Additional anion exchange chromatography steps may be used in the further purification as is disclosed in greater detail hereinbelow.

In yet another aspect, the invention is a purified recombinant TNF composition having a TNF content of at least 95% as determined by SDS-PAGE analysis, an endotoxin content of less than about 0.1 nanograms/mg, said TNF being substantially free of pyrogens as determined by the USP rabbit pyrogen test at a dosage range of 1.0 to 2.4 x $10^5$ U/Kg. The TNF produced is substantially similar to mature TNF or may contain modifications to the molecule, particularly N-terminal sequence deletions and substitution in amino acids.

In another aspect, the invention relates to a process for obtaining a partially purified TNF under pH conditions that reduce hydrolysis of the TNF. The pH is controlled so that it is greater than 5.5 during the first stage of the process in which TNF-producing cells are disrupted, the cell debris is removed therefrom, and the remaining fluid is diafiltered through a hydrophobic porous matrix, preferably a continuous hydrophobic porous matrix, to produce a filtrate.

Figure 1 shows an elution profile from DEAE Sepharose of a crude bacterial extract containing recombinant TNF.

Figure 2 shows the elution profile of hydrophobic HPLC using a phenyl-TSK column, to which TNF has been absorbed, using a decreasing salt concentration gradient.

Figure 3 also shows the amino acid sequence of mature TNF, but with a number of possible muteins, including N-terminally deleted muteins.

Figure 4 is a flow diagram of a preferred TNF purification process. Optional steps in the preferred process are denoted by a broken line.

Figure 5 is a flow diagram of an alternative preferred TNF purification process.

As used herein, the term "tumor necrosis factor" (TNF) refers to a molecule that is substantially equivalent to the amino acid sequence of Figure 3 and is capable of selective cytotoxicity against tumor cells. Such selective cytotoxicity, according to the definition herein, is demonstrated by activity in the in vitro cytotoxicity assay based on the continuous murine connective tissue cell line L-929 as described in PCT Publication WO 86/02381, published April 24, 1986 of Cetus Corporation.

This activity is confirmed by in vitro cytotoxicity assay against human tumor cells, i.e. the cytotoxicity against L-929 appears to generalize to human tumors. In vivo assays can also be used if desired to confirm these results.

The amino acid sequence of TNF is shown in Figure 3. The sequence of Figure 3 represents the mature or native form of human TNF. A "substantially equivalent" amino acid sequence of TNF means the amino acid sequences are identical or differ by one or more amino acid alterations (deletions, additions, substitutions) that do not cause an adverse functional dissimilarity between the altered or mutein form of the protein and native form. "Adverse functional dissimilarity" is manifest by an altered form of TNF if, in purified form, its activity in the L-929 in vitro cytotoxicity assay is destroyed. Further, individual amino acid residues in the protein may be modified by oxidation, reduction, or other derivatization, or the protein may be cleaved to obtain fragments that retain activity. Such alterations that do not destroy activity in the L-929 in vitro cytotoxicity assay mentioned above do not remove the protein sequence from the definition of TNF.

The specific nature of TNF protein depends on the pH of its environment, if suspended or in solution, or of its environment when crystallized or precipitated, if a solid, and thus may be in the form of pharmaceutically

acceptable salts or may be in neutral form. The free amino groups of the protein are, of course, capable of forming acid addition salts with, for example, inorganic acids such as hydrochloric, phosphoric, or sulfuric acid; or with organic acids such as, for example, acetic, glycolic, succinic, or mandelic acid. The free carboxyl groups are capable of forming salts with bases, including inorganic bases such as sodium, potassium, or calcium hydroxides, and such organic bases as piperidine, glucosamine, trimethylamine, choline, and caffeine. In addition, the protein may be modified by combination with other biological materials such as lipids and saccharides, or by side chain modification such as acetylation of amino groups, phosphorylation of hydroxyl side chains, or oxidation of sulfhydryl groups. The TNF recombinantly produced using bacteria as hosts presumably lacks these additional moieties. All of these modifications are included within the scope of the definition. so long as the TNF activity is retained.

It is also understood that minor modifications of primary amino acid sequence may result in proteins which have substantially equivalent or enhanced activity as compared to the sequence set forth in Figure 3. These modifications may be deliberate, as through site-directed mutagenesis, or may be accidental such as through mutation in hosts which are TNF producers.

Within the foregoing definition of TNF are analogs of TNF specifically or randomly altered wherein the altered forms exhibit the selective cytotoxicity mentioned hereinabove. Such active TNF analogs or muteins may exhibit improved properties such as increased potency in cytotoxicity assays, greater homogeneity when produced by a recombinant host, or improved processing characteristics in purification. Such TNF analogs may also have additional functionalities not obtained in the native form, e.g., conversion of a cysteine residue leaving a free sulfhydryl in the unaltered cysteine residue that may be used to couple TNF to other moieties by formation of a disulfide or thioether bond.

Forms of TNF that are inactive in the in vitro cytotoxicity assay mentioned above may also be formed by random, site-specific or deletion mutagenesis. Although such forms do not fall within the definition of TNF herein. such forms may be useful for purposes other than causing cytotoxicity to tumor cells or direct therapy of patients having tumors susceptible to cytotoxic properties of TNF. Such forms of TNF may still potentiate or synergize the activity of other active lymphokines, for example, interleukin-2 and gamma interferon. Forms of TNF inactive in the in vitro cytotoxicity assay mentioned above may nevertheless be purified by the processes disclosed herein.

Specific examples of TNF analogs include N-terminally deleted species of the protein, including those having deletions of the N-terminal 1, 2, 3, 4, 5, 6, 7, 8, 9, 10, 14, and 31 amino acids as shown in Figure 3. Muteins lacking up to and including the first ten amino acids at the N-terminus have been found to have comparable or greater specific activities as compared to the TNF of the sequence shown in Figures 3 and 4. The pattern of specific activities appears to follow a bell-shaped curve with an optimum activity when 6-8 N-terminal amino acids are deleted. Accordingly, the definition of TNF specifically includes these truncated forms, as clearly, such deletions do not destroy, but, in fact, sometimes enhance biological activity.

Also included are species of TNF in which any or all of the cysteine residues of the TNF molecule have been converted to serine or other neutral amino acids, for example, glycine or alanine. In general, neutral amino acid replacements of the cysteine at position 69 result in active TNF proteins. It appears that the cysteine at position 101 is also dispensible, and muteins having alternative neutral amino acids in this position, as well as muteins wherein both cysteines 69 and 101 have been replaced, have been prepared. These muteins can also be modified to obtain truncated forms, for example to lack 1-10 amino acids at the N-terminus, sequences of amino acids at the C-terminus, or both. These muteins also retain TNF activity and may have enhanced biological activity in vitro and in vivo.

As to notation, for convenience, the protein having the amino acid sequence numbered 1-157 in Figure 3 will be used as a reference and designated herein mTNF (mature TNF). All other amino acid sequences having homology with mTNF and showing TNF biological activity are referred to as "muteins" of mTNF and are denoted as to their differences from mTNF using the numbering of residues shown in the figure. For example, muteins which have substitutions for cysteine at position 69 will be denoted using the substituted residue and the position number, e.g., peptides having a serine in place of the cysteine at position 69 are designated $ser_{69}$ TNF. If a residue is simply missing. it is renamed as a des-residue so that, for example, the mutein wherein the serines at positions 3 and 4 are deleted is designated des-$ser_3$des-$ser_4$ TNF. Muteins which lack segments of amino acids at the N- or C-terminus are denoted according to the terminus affected. N-terminus deletions lacking a number of amino acids are denoted by $\nabla$, followed by the number of amino acids missing. For example. muteins which lack one N-terminal amino acid as compared to the protein shown in Figure 3 are designated $\nabla$1TNF. For deletions at the C-terminus. the $\nabla$ will be followed by the number of the last remaining residue and a minus sign. Thus, for the mutein having 7 amino acids removed from the C-terminus, the designation would be $\nabla$150-TNF. Where combinations of the foregoing alterations are made, the designation shows all of them, e.g., $\nabla$1des-$ser_3$des-$ser_4$$ser_{69}$$\nabla$150-TNF.

Not all muteins of TNF are recombinantly or deliberately produced. Indeed, the sequence obtained for the 22 N-terminal amino acids of the HL-60 secreted TNF contains minor modifications in the primary structure, although both native and recombinant proteins exhibit TNF activity. Specifically, the recombinant sequence has an additional pair of serine residues preceding the serine at position 5 before resuming the homology between positions 4-12 of the HL-60 derived protein and positions 6-14 of the deduced sequence.

As used herein, the term "TNF" is intended to include multimeric forms. TNF is known to form aggregates or multimers, predominantly dimers. Such multimers are selectively cytotoxic and are suitable for in vivo use. The

TNF produced by the processes according to the invention is substantially a homogeneous composition of monomeric TNF on SDS-PAGE electrophoresis.

As used herein, "chromatography" means that a subject mixture is treated with an adsorbent or other support matrix and then eluted, usually with a gradient or other sequential eluant, as opposed to a simple one step process. Material eluted from the support matrix is designated eluate. The sequential elution is most commonly done by placing the support matrix in a column and supplying an eluting solution which changes its character either stepwise or preferably by gradient. However, other methods may be used, such as placing the support matrix in a filter and sequentially administering eluants of differing character.

As used herein, "by chromatography" means that this elution must be done so as to elute less than all of the materials retained by the support matrix at one time. Thus, if done batchwise, the elution must be accomplished so as to selectively remove only the desired component while leaving the remaining materials retained by the support matrix or must comprise more than one step in which, for example, the desired component is selectively removed by first eluting the undesired component, followed by eluting the desired component retained by the support matrix.

The term "continuous hydrophobic matrix," as used herein, is intended to encompass hydrophobic membranes. Such membranes are exemplified by polymers of lower alkylenes and substituted alkylenes. Polypropylene and polyethylene are examples of the former. Polytetrafluoroethylene is an example of the latter.

By "porous" is meant that the membrane has openings therein that substantially traverse the membrane. In short, a porous membrane has holes in it that go through the membrane. Such porous membranes can filter materials, allowing some portion of the materials to pass through the membranes and retaining the remaining portion. "Porous" as used herein is not intended to mean pitted, i.e., having openings that do not go through the membrane.

As used herein "hydrophobic interaction matrix" means an adsorbant that is a hydrophobic solid such as polystyrene resin beads, rubber, silicon coated silica gel, or cross linked agarose sufficiently substituted with hydrophobic functional groups to render the material hydrophobic. Alkyl substituted agarose and aryl substituted agarose such as phenyl or octyl agarose are examples. Such alkyl and aryl substituents are referred to herein as hydrocarbyl. Materials to be chromatographically separated on a hydrophobic interaction chromatography (HIC) matrix are first sorbed to the HIC matrix in a high salt solution and are desorbed from the HIC matrix by elution in a low salt concentration solution or a hydrophobic solvent such as a polyol. The term "hydrophobic non-silica interaction matrix" refers to the matrix which does not contain silica. Therefore, it is a hydrophobic chromatographic support comprising a non-silica matrix with hydrophobic groups bound thereto.

As used herein "anion exchange matrix" means a solid or gel support matrix that is charged in aqueous solutions. The support matrix may be agarose sufficiently substituted with amine functional groups to have a net charge in aqueous solutions. The material to be sorbed is bound to the anion exchange matrix in a low salt solution and is eluted from the anion exchange matrix in a high salt eluant containing anions such as chloride ion which bind to the anion exchange matrix and displace the sorbed material.

As used herein, "mixture," as it relates to mixtures containing TNF, refers to a collection of materials that includes TNF, but that also include other proteins. If the TNF is derived from recombinant host cells, the other proteins will ordinarily be those associated with the host. Where the host is bacterial, the contaminating proteins will, of course, be bacterial proteins. Furthermore, if the bacterial host is Gram-negative, endotoxins or lipopolysaccharide may be present. These endotoxins are routinely removed in the purification processes according to the invention. However, if the TNF is associated with native sources, such proteins will be mammalian. Other non-proteinaceous materials may also be present, but generally do not constitute a purification problem.

By "high salt concentration conditions" is meant an aqueous solution wherein an ionic substance is present to create conditions of high ionic strength. Ionic strength is defined, as is understood in the art, to be calculated from the putative concentrations of the various ions placed in solution modified by their activity coefficients. Workable high salt concentrations are typified by solutions containing high concentrations of ammonium sulfate. However, other salts such as sodium chloride, potassium chloride, sodium sulfate, sodium nitrate, or sodium phosphate can be used instead, provided solubility permits, and provided the same ionic strength can be obtained.

As used herein, the term "host" refers to a cell producing TNF. Such host cells may be mammalian cells that produce TNF from DNA sequences coding for TNF that are endogeneous to the genome of the cell in its native state. Preferably, the host cell will be a recombinant host cell, i.e., one into which a TNF-encoding DNA sequence has been introduced by means of recombinant molecular biological methods. Such a host cell within the definition includes eukaryotic hosts, including, for example, such mammalian cells as mentioned above into which, in addition, a TNF-encoding DNA sequence has been introduced.

Alternatively, the host cell will be a eukaryotic microorganism such as a yeast or fungus into which the DNA sequence encoding TNF has been introduced. Most preferred are prokaryotic host cells, such as members of the genuses Bacillus, Streptomyces, and Escherichia. Among Bacillus hosts, Bacillus subtilis is preferred. Within the genus Escherichia, E. coli is preferred.

The term "diafiltration and diafilter," as used herein, refers to a filtration process wherein the material to be filtered is maintained in a volume of liquid. Solid retained by the filter is designated retentate; liquid material

passing through the filter is designated filtrate. In diafiltration processes, as the liquid filtrate is removed from the retentate across the filtering medium, liquid volume is replaced on the retentate side of the filter, preferably at a rate equal to the rate filtrate is removed. As a result, material that is capable of passing through the filter is washed from the retentate.

As used herein, the term "biologically active proteins that are hydrophobic at physiological pH" refers to proteins that are hydrophobic, but soluble in a pH range between about 7.2 and 7.6. Such biologically active proteins are typified by recombinant TNF and recombinantly produced ricin toxin A chain and have the characteristic of binding to hydrophobic supports such as phenyl-TSK and phenyl agarose.

One of the purification methods herein is the application of a mixture containing TNF to a hydrophobic non-silica interaction matrix. A number of hydrophobic interaction matrices that do no contain silica are known, and include, for example, phenyl-TSK, a resin commonly used as an HPLC support column. Appropriate hydrophobic supports, in general, are comprised of alkyl, phenyl, or other essentially hydrocarbyl substituents of sufficient hydrocarbon content to be hydrophobic, bound to a polymer matrix, usually a carbohydrate. Other hydrophobic polymers include polyacrylamide and polyolefins. Other exemplary hydrophobic supports which are usable in this method of the invention include phenyl sepharose, octyl sepharose, phenyl agarose, and octyl agarose. Phenyl-TSK is preferred in this particular method of the invention.

In a preferred mode, the mixture containing TNF protein is brought to around 1.5- 2 M ammonium sulfate, preferably 1.8 M ammonium sulfate contained in approximately 0.1 M sodium phosphate, approximately pH 7.0. Of course, other buffers maintaining approximately neutral pH could be used.

The TNF protein elutes from a hydrophobic column at a low salt concentration depending on the mutein form chromatographed and on whether analytical or preparative columns are used. As a preliminary matter, it is noted that for analytical columns, ∇4TNF, for example, is still retained when the buffer concentration remains at approximately 0.1 M even when the concentration of ammonium sulfate is reduced to zero. However, during the subsequent decreasing ion gradient, when the buffering ions are also deleted from the solution, the ∇4TNF elutes at approximately 0.02 M sodium phosphate. Accordingly, ∇4TNF elutes from the hydrophobic analytical support in an ionic strength range corresponding to approximately 0.02 M sodium phosphate, an ionic strength that can be mimicked by appropriate concentrations of other salts. On the other hand, mTNF elutes from the phenyl-TSK analytical column when the ammonium sulfate concentration is reduced to about 0.4 M, and the buffer is still present.

Table 1 shows the conditions for elution of the various TNF muteins from a preparative phenyl-TSK support. The results differ from those obtained on an analytical column, as is often the case for protein separations. However, the muteins also in this case exhibit different behaviors from each other. Their behavior in preparative column is, of course, of greater relevance to the use of hydrophobic interaction matrices in purification.

Table 1

Phenyl-TSK Elution Gradient for Preparative Columns

| TNF Mutein | % TNF Mutein Eluted at 0.36M $(NH_4)_2SO_4$ 100 mM $NaP_i$ | 0.0M $(NH_4)_2SO_4$ 100 mM $NaP_i$ | 0.0M $(NH_4)_2SO_4$ 20 mM $NaP_i$ |
|---|---|---|---|
| mTNF | 80% | 0% | 20% |
| ∇4TNF | 80% | 0% | 20% |
| ∇7TNF | 25%* | 5%* | 70%* |
| ∇6TNF | 50% | 0% | 50% |
| ∇8TNF | 50% | 0% | 50% |
| ∇9TNF | 30% | 0% | 70% |
| ∇10TNF | <1% | 10% | 90% |

*Each sample showed the same IEF pattern, amino acid composition, and N-terminal sequence.

The mixture subjected to this process of the invention is preferably a mixture from bacterial culture wherein the contaminants are bacterial proteins. In a typical such preparation, the bacterial host transformed with vectors encoding recombinant TNF is cultured and induced for TNF production according to the sequences controlling the gene. Typical vectors and means of expression are set forth in detail in PCT Publication No. WO86/02381 cited above.

The crude extract is prepared, for example, by sonicating the bacterial host expressing the recombinant TNF gene. It is preferred that a preliminary purification of this extract on anion exchange support be performed. The various TNF muteins also show differing elution patterns in anion exchange chromatography. For example, using DEAE cellulose and an increasing NaCl gradient, mTNF was eluted at 40 mM NaCl, ∇4TNF began to elute late in the 40 mM NaCl portion and mainly came off the column at 60 mM, ∇10TNF eluted at 60 mM NaCl, and ∇6-, ∇8-, and ∇9TNF eluted at 80 mM NaCl (both from DEAE cellulose and from Mono Q).

Briefly, the appropriate E. coli strain, in the illustrated instances, E. coli strain K12 MC1000 lambda lysogen (ATCC No. 39,531 available from the American Type Culture Collection, Rockville, MD, USA) or a similar strain such as DG95, is transformed with a recombinant vector effective in expressing the desired TNF. In the illustrated vectors, all of the gene sequences are under the control of the $P_L$ promoter and the cells are grown at 37°C under standard growth medium conditions to an $OD_{600}$ of about 0.5 and then induced by increasing the temperature to 42°C. After two hours, the cells are sonicated and the sonicate is verified to contain TNF activity using in vitro cytotoxicity assay employing murine L-929 cells. Of course, alternative expression vectors and alternative hosts could be employed to generate the recombinant TNF. Other bacterial control sequences, such as the tryptophan (trp) promoter system (Goeddel et al., Nucleic Acids Res. (1980) 8:4057) or the penicillinase or lactose (lac) promoter systems (Chang, et al., Nature (1977) 198:1056) could also be used with appropriate conditions for growth and promoter induction. Other bacterial hosts besides E. coli include, for example, various species of Bacillus or Pseudomonas. In addition, eucaryotes such as yeast, or cells grown in tissue culture such as Vero, HeLa, or CHO cells, could be used if appropriate vectors are constructed. Such techniques for expression of foreign genes in a variety of hosts are understood in the art.

The desired TNF will be accumulated in the cell or secreted into the medium, depending upon the construction of the expression vector, to include or not to include a functional leader sequence. If a signal sequence is included and the TNF is secreted into the medium, the starting material comprises the supernatant after the cellular debris has been removed. If the TNF is accumulated in the cells, the cells are disrupted, such as by sonication or by mechanical means such as a Hughes press to liberate the contents. The cellular extract of harvested supernatant is then used as the initial mixture containing TNF.

In a preferred mode of carrying out this method of the invention, the starting material is first subjected to ion exchange chromatography using, for example, DEAE agarose, DEAE cellulose or QAE agarose, preferably DEAE agarose. The treatment conditions are such that the TNF is retained on the support and can be eluted by

increasing ionic strength. Typical conditions are Tris buffer at pH 7-9, preferably around pH 8, and a salt concentration of approximately 1 mM NaCl. Elution is accomplished by increasing the salt concentration in either stepwise or gradient increments to obtain elution of the retained components; TNF elutes under these conditions at approximately 55 mM NaCl. The TNF-containing fractions are determined by subjecting the fractions collected to cytotoxicity or other assay for TNF, and those containing TNF are pooled and subjected to further purification.

In this method of the invention, the TNF-containing mixture, preferably the pooled fractions from the DEAE Sepharose column, is brought to 1.8 M ammonium sulfate, or the ionic strength of solution is comparably adjusted with alternate salts in the presence of 0.1 M sodium phosphate buffer. The solution is then applied to a chromatographic column containing the hydrophobic support, preferably phenyl-TSK support. The TNF is retained under these conditions. The column is then eluted by decreasing the concentration of ammonium sulfate, and finally the concentration of phosphate in the buffer. The process is most efficiently conducted under high performance (HPLC) conditions, but this is not a requirement. Again, the eluted fractions are tested for TNF activity using any convenient assay such as the cytotoxicity assay of PCT Publication WO86/02381 cited above. Those fractions which contain TNF activity may then optionally be further purified by gel filtration with a pore size adjusted according to peculiar size of the contaminants. Any gel filtration resin with a size range between that of S-200 and G-25 may be used to separate the desired TNF from the salts remaining from the hydrophobic chromatography support step.

The resulting TNF preparation is homogeneous by SDS-PAGE and recoveries are typically 50-80% of total activity.

One alternative process for purifying TNF according to the invention comprises two stages as shown in the flow diagrams of Figures 4 and 5. In the first stage of this process, TNF is partially purified from a fluid containing TNF and other cell products. This TNF-containing fluid, obtained from collected disrupted TNF-producing host cells by removing the cell debris of the disrupted host cells, is filtered through a substantially continuous hydrophobic porous matrix to produce a filtrate containing a mixture enriched in TNF. To enhance the yield of the TNF in the process, the pH conditions of the collected TNF-producing cells, their disruption and the removal of cell debris are controlled to reduce hydrolysis of the TNF. The pH conditions of the filtration of the TNF-containing fluid may be similarly controlled to maximize the TNF yield.

In a preferred embodiment of this process for purifying TNF, TNF-producing host cells are collected in a volume of fluid and the pH of the collected host cells is adjusted or maintained at a pH that reduces hydrolysis of TNF. In all of the subsequent steps of the first stage, the pH of the material produced from the host cell is similarly adjusted. The collected host cells are disrupted and disrupted cell debris is removed leaving a fluid containing TNF and other cell products. This TNF-containing fluid is then passed through a continuous porous hydrophobic matrix to produce a filtrate containing a mixture enriched in TNF or a partially purified TNF.

In the second stage of the process, the mixture enriched in TNF is subjected to at least two chromatographic steps, one on a hydrophobic interaction (HIC) matrix and one on an anion exchange matrix. In one embodiment of the process, illustrated in Figure 4, the mixture is first chromatographed on an HIC matrix and selectively eluted therefrom. The eluate so produced is substantially free of residual proteins and nucleic acid degradation products such as nucleotides and nucleosides. The material selectively eluted from the HIC matrix is then further chromatographically purified on an anion exchange matrix. Optionally, the material selectively eluted from the HIC matrix is desalted prior to this anion exchange chromatography step. A fraction high in TNF is selectively eluted from the anion exchange column using an appropriate salt. Optionally, depending upon the type and amount of salt solution used to elute the fraction high in TNF from the anion exchange matrix after the chromatography on the HIC matrix, the fraction high in TNF may require desalting on an appropriate material. In an additional option, illustrated in Figure 5, prior to chromatographing the mixture on the HIC matrix, the mixture is chromatographed on an anion exchange matrix, and eluted therefrom with an appropriate salt solution.

The filtrate containing a mixture of TNF or partially purified TNF produced by the first stage of the process represents at least about 20% of the theoretical yield of TNF produced by the host cells. More typically the partially purified TNF amounts to between 40 and 50% of the theoretical yield of TNF produced by the cells. The mixture comprises from 40% to 70% TNF as a fraction of total protein recovered. The endotoxin level in the mixture contains between about 10 ng/ml and 10µg/ml of the mixture.

The purified TNF recovered at the end of stage 2 of the process has a TNF content of at least 95% as determined by SDS-PAGE analysis and an endotoxin level of less than 0.1 ng/mg TNF. TNF level frequently exceeds 98% and may exceed 99%. In addition, the material is substantially free of pyrogens as determined by the USP rabbit pyrogenicity test at a dosage in a range between 1 x $10^5$ and about 2.4 x $10^5$ U/Kg.

In the process according to the invention, the host cell may be any one of a TNF-producing mammalian cell, a recombinant TNF-producing eukaryotic cell, including a recombinant mammalian cell, a recombinant eukaryotic microorganism such as a yeast, for example, Saccharomyces, or other fungus such as those of the genus Aspergillus, a recombinant prokaryotic microorganism such as the Gram-positive microorganism of the genus Bacillus, including B. subtilis or B. cereus, or genus Streptomyces or Gram-negative microorganism such as the genus Escherichia, Serratia or the like. E. coli is particularly preferred.

The host cells, preferably E. coli transformed with a plasmid having a DNA sequence encoding TNF which is expressed by the host cell to produce TNF, are grown in a suitable growth medium to a desired cell density typically measured by optical density (OD) at 680 nm. Optical densities between about 20 and 40 are typical

and an OD of about 30 is preferred.

For expression of TNF in E. coli transformed with a plasmid expression vector that expresses TNF under the control of most bacterial promoters, E. coli strain MM294, (Talmadge, K., et al., Gene (1980) 12:235 and Messelson, M., et al., Nature (1968) 217:1110), is used as the host. An appropriate MM294 strain has been deposited in the American Type Culture Collection under accession number 39,894 on October 19, 1984. In such expression vectors under control of the tryptophan (trp) promoter, the trp concentration in the medium is carefully controlled to become limiting at the desired host cell density at the time TNF expression is desired.

For expression under the control of the $P_L$ promoter and gene N ribosome binding site, E. coli strain K12 MC1000 lambda lysogen $N_7N_5CI857SusP_{80}$, ATCC accession number 39,531 is used. Expression of TNF under control of the $P_L$ promoter is obtained by shifting the temperature from 30 to 42°C when the desired OD of the culture is obtained.

The composition of the growth medium will, of course, depend upon the particular microorganism used. In general, an aqueous medium is used that contains assimilable sources of carbon and nitrogen, energy sources, magnesium, potassium and sodium ions, and such amino acids and purine or pyrimidine bases as required.

After the cells are harvested from the culture medium, they may be concentrated, if necessary, by filtration, centrifugation or other means. If TNF is produced as an intracellular product, it is preferred to remove from the harvested cells substantially all of the culture medium by washing the cells in an appropriate solution adjusted for pH and osmolarity so as not to prematurely break the cells or hydrolyse the desired protein product. Various means may be used to wash the cells, including low speed centrifugation so as not to shear the cells, alternating with cell washing or diafiltration. Diafiltration is preferred.

The pH of the concentrated cell suspension is also adjusted in a range that reduces hydrolysis of the TNF to be purified. pH adjustment is carried out by adding sufficient amounts of acid, preferably HCl, or base, preferably NaOH, at a concentration sufficient to reach the desired pH without degrading the protein or cells. A pH range between 5.5 and 9.5 is maintained. For TNF purification, alkaline pH is preferred and a pH of 8.5 is most preferred.

The cell membranes of the collected host cells are disrupted to release intracellular TNF. The choice of cell disruption methods will depend largely on the amount of cells harvested, but any conventional technique will be sufficient. Conventional cell disruption techniques such as homogenization, sonication, or pressure cycling may be used. Preferred methods are sonication or homogenization with a cell homogenizer such as a Manton-Gaulin homogenizer. Cell disruption should break substantially all of the cells so that effectively no intact cells remain in the processing of the material during subsequent steps. The end point of the disruption step may be determined by monitoring the drop in the optical density of the disrupted cells to an optical density of between about 65% and 85% of the initial OD. The pH of the disrupted cell, or disruptate, will also be monitored and adjusted as necessary. pHs between 5.5 and 9.5 are acceptable. Alkaline pHs within the range are preferred and a pH of 8.5 is most preferred. pH adjustment of the disruptate may be made with suitable buffered pH solutions.

To further reduce TNF hydrolysis, it is desirable to carry out the disruption with temperature control at a temperature between 0 and 10°C. A temperature between 0 and 4°C is preferred. Subsequent to the disruption of the host cells, the particulate matter is separated from the liquid phase of the disruptate by any conventional separation method. The removal of particulate matter at this point in the purification process is desirable because it has been discovered that cellular components associated with the cell debris can cause hydrolysis of the TNF produced by the cell.

Various means are known for removing particulate matter from the cell disruptate. Flocculating agents, such as calcium ion supplied from dissolved calcium chloride, may be added to the disruptate to aggregate suspended particulate matter. The aggregated particulate matter in cell debris is removed by centrifugation or settling. Various appropriate flocculating agents, such as polyelectrolytes, or caking agents, may be used. Various flocculating agents will be known to those skilled in the art. Centrifugation, without the use of flocculating agents, may also be used to accumulate and separate the liquid phase from the particulate matter of the disruptate.

Alternatively, the particulate matter may be separated from the liquid phase of the cell disruptate by filtering through a non-hydrophobic filter, preferably a hydrophilic cellulose ester membrane. The pore size of the filter will be selected so as to optimize the flux of the liquid phase across the filter while retaining the particulate matter. Thus, the optimal pore size will depend upon the extent to which the cell material has been disrupted and whether the cell debris have been aggregated through the use of flocculating agents. Thus, appropriate pore sizes may range from 0.01 microns to 2 microns. Pore sizes between .01 and 1 microns are preferred. Prior to separation of the particulate matter from the liquid phase of the disruptate, the pH of the disruptate is once again adjusted to between pH 5.5 and 9.5. Alakline pH is preferred and a pH of 8.5 is most preferred. If filtration is the method used for separating the liquid phase from the particulate matter of the cell disruptate, cross-flow filtration is preferred.

Following separation of the liquid phase of the disruptate from the particulate matter, the liquid phase is filtered through a substantially continuous porous hydrophobic matrix. Such hydrophobic substantially continuous matrices may be made of, for example, polymers of lower alkylenes and substituted polyalkylenes such as polypropylene and polyethylene. Polytetrafluoroethylene (PTFE) is preferred. The substantially continuous porous hydrophobic matrix will generally be in the form of a membrane having a defined pore size.

Such membranes may be obtained from Dorr-Oliver. Inc., Stamford, Connecticut and W. L. Gore & Associates, Inc., Newark, Delaware. It has been found that by varying the pH, ionic strength, membrane symmetry and membrane pore size, it is possible to elute the TNF selectively and permit a small subset of proteins, including TNF, to pass through the membrane, and thereby accumulate a filtrate enriched in TNF. A pH between 5.0 and 9.5 is desirable for the filtration of TNF through the hydrophobic membrane. pHs in the acidic portion of the range are preferred and a pH of 5.5 is especially desirable. In general, any buffering system may be used so long as it can maintain the pH in the desired range. Such buffers include, for example, acetate, citrate and succinate. Ten millimolar (mM) acetate buffer has been used to good effect. Pore sizes sufficiently large to pass proteins of about 100,000 molecular weight may be used. Pore sizes from 0.1 to 3.0 microns are acceptable and a 1.0 μ pore size is preferred.

Various filtration geometries may be used in filtering the liquid phase of the cell disruptate through the hydrophobic membrane. For large scale purifications, it is preferred to use diafiltration.

The material obtained after the filtration of the material through the hydrophobic membrane, at the end of the first stage of the purification process. is characterized by having a large content of TNF. Based on biological activity, approximately 50% of the TNF produced by the host cell can be recovered in the filtrate from the hydrophobic membrane. Of the protein in the filtrate, 40 to 60% of the total protein has been identified as TNF. This represents a four to six-fold purification of the TNF through the first stage of the purification process. In addition, endotoxin levels, as determined by limulus amebocyte lysate tests, range from 10 nanograms to 10 micrograms per ml of filtrate.

The steps of the recovery process subsequent to the first stage of the purification process are designed to separate the TNF from E. coli protein to a high level of purity, preferably at least about 95%, and more preferably at least about 98%. Simultaneously, these purification processes also reduce the levels of pyrogenic substances exogenous to TNF to a level acceptable for parenteral administration to patients. Such pyrogenicity as is detectable is believed to be inherent in the TNF molecule.

The subsequent steps in the purification of TNF are chromatographic purification steps as defined hereinabove. In an optional step the filtrate of TNF and proteins obtained at the end of the first stage is concentrated. The filtrate of TNF and protein may be concentrated by chromatography on an anion exchange matrix. The mixture is adjusted to a pH appropriate for use on the anion exchange matrix in an acceptable buffer. In general, mild alkaline pH in a range between 7.5 and 8.5 is preferable, and a pH of 8.2 is most desired. Appropriate buffers include tris(hydroxymethyl)aminoethane, glycylglycine, and triethanolamine. Ten mM Tris is preferred. Alternatively the filtrate of TNF and proteins obtained at the end of the first stage may be concentrated by ultrafiltration using a filter of a pore size sufficiently small to prevent TNF from passing through the filter. A pore size sufficient to retain proteins of molecular weight above 10,000 daltons is adequate for this purpose. In addition, the filter must be made of a material to which the TNF does not significantly adsorb so that the TNF remains substantially in the retentate. A mildly hydrophobic membrane is generally acceptable. Polysulfone membranes are preferred.

In addition to concentrating the filtrate of TNF and protein, the anion exchange matrix may be selected from those that are able to selectively remove nucleic acid degradation products such as nucleotides and nucleosides by permitting the TNF from the mixture to be selectively eluted from the anion exchange matrix in a filtrate that is substantially free of nucleic acid. Among the appropriate anion exchange resins are those that contain bound tertiary and quaternary ammonium ion. Such anion exchange matrix material is typified by QAE-agarose, QAE-cellulose, and DEAE-agarose. The mixture is loaded on an anion exchange matrix. Prior to eluting the TNF from the anion exchange matrix, the matrix is washed with a buffered salt solution, for example, NaCl. The NaCl wash selectively elutes the nucleic acid degradation products from the anion exchange matrix while selectively retaining TNF on the column at the molar concentration of the salt wash. Molar anion strengths of the wash will generally be less than 65 mM.

After the column is washed, the TNF is eluted from the anion exchange matrix in an appropriate increasing gradient of anion that is applied to the column. The eluate is monitored by optical means for absorbance in a range of 280 nm to detect the fractions of eluate containing protein as the fractions elute from the column. The protein is eluted from the column in a salt gradient that ranges between 65 at 300 mM. Linear and stepwise gradients may be used to advantage in the process. Chloride is the preferred anion, although phosphate ion and sulfate ion are acceptable.

Provided that sufficient amounts of nucleic acid degradation products and protein have been removed in the first stage of filtering the TNF-containing fluid through the hydrophobic porous matrix, it may be desirable to dispense completely with the initial optional second stage purification step using the anion exchange column. In either event, the filtrate from the hydrophobic membrane, or the eluate from the anion exchange column, is next chromatographed on a continuous hydrophobic porous interaction matrix as described above for the first method for TNF purification, except that the matrix may also contain various silicas, which are, in general, of sufficient hydrophobicity to serve as a hydrophobic interaction matrix. In general, any hydrophobic material that will bind TNF under high salt conditions within the pH range of from 4 to 9 may be used, although those that bind in a pH range between 5 and 9 are preferred. Preferred for this particular method are alkyl agaroses as the hydrophobic interaction matrix. Phenyl agarose is particularly preferred, although octyl agarose may also be employed. The most advantageous form of alkyl agarose is one in which agarose content is between about 3.5 and about 8%, and is crosslinked. More preferred are phenyl aragose resins in which the agarose content is between 4 and 6% and crosslinked. Most preferred is a phenyl agarose having 6% crosslinked

agarose.

Prior to loading the column with the mixture or filtrate, the column is equilibrated with a high salt solution as described above for the first method. Workable high salt concentrations are typified by solutions containing high concentrations of ammonium sulfate. Other salts such as sodium chloride, potassium chloride, sodium phosphate, sodium sulfate, magnesium sulfate, and sodium nitrate, can be used provided that solubility permits, and provided that the same ionic strength can be obtained. In a preferred mode, the column is equilibrated with ammonium sulfate, in a range between 1.5 and 2 molar. Preferably, the sodium sulfate is used at a concentration of 1.8 molar. Four molar sodium chloride can also be used.

The eluate or filtrate is brought to high salt concentration as defined above and is loaded on the column. In general, the high salt concentration is achieved by adding ammonium sulfate to between 1.5 and 2 molar, preferably 1.8 molar. The pH of the high salt solution is maintained between about 5 and 7. A pH of 5.5 is preferred. The column and the material bound thereto at this high salt concentration is maintained at a temperature in a range between 0 and 25°C. It is generally preferred that the material and column be maintained at a temperature well below 25°C during this step, with 4°C being preferred.

The TNF is eluted from the hydrophobic interaction matrix at a low salt concentration. The particular salt concentration will depend upon the mutein form of the TNF molecule chromatographed and on the particular hydrophobic interaction matrix used. In general, the TNF material elutes as the salt concentration drops. The particular salt concentration at which the TNF elutes will also depend somewhat upon the buffer used.

Various materials may be used to elute the TNF from the column, including various chaotropic agents and nonionic detergents. Polyols may also be used so long as they remain substantially flowable at the temperature at which the column is maintained. Propylene glycol and ethylene glycol may be used. A linear gradient of ethylene glycol in an appropriate buffer in a range of from 0 to 60% ethylene glycol is preferred. As indicated above, the buffer may vary and the pH may range between 5 and 8. Four mM acetate buffer may be used at a pH of about 5. A pH of 5.5 is preferred.

Optionally, the material eluted from the hydrophobic interaction matrix may be desalted. Preferably, the material is desalted using a size exclusion resin such as G-10, G-15 or G-25 Sephadex.

Once eluted from the hydrophobic interaction matrix, the protein solution or optionally desalted protein solution is chromatographed on an anion exchange matrix. Any anion exchange matrix can be used which will selectively bind the protein and allow the chaotropic agent or detergent to pass so that the bound protein may be eluted. Such anionic exchange matrices are well known to those skilled in the art and in general comprise substituted amines in an agarose or cellulose matrix. Trisubstituted and quaternary substituted amines are particularly preferred. Diethylaminoethyl (DEAE) agarose is one such ionic exchange matrix. Quaternary substituted agarose and cellulose are also suitable. A convenient quaternary ammonium anionic exchange medium is a bound quaternary ammonium ion containing matrix.

As mentioned above, prior to loading the protein solution eluted from the hydrophobic interaction matrix onto the anion exchange matrix, the protein solution may be optionally desalted. If the protein solution eluted from the hydrophobic interaction matrix is not desalted, then the eluate is diluted with double distilled deionized water to decrease the ionic strength of the solution so that the protein will bind to the column. Ionic strength of the material is determined by monitoring the conductivity of the solution so that it is below 3 millisiemens (mS). In general, a conductivity of approximately 2 mS is preferred. The pH of the solution is adjusted to between about 7 and 9. A pH of 8 is preferred. Prior to loading the pH-adjusted eluate on the column, the column is equilibrated with buffer. Sodium phosphate, Tris sulfate or Tris chloride are appropriate. Ten mM is preferred.

A salt gradient is used to elute the TNF protein selectively from the column. A sodium chloride or sodium sulfate gradient may be used. The salt gradient is buffered with an appropriate buffer which is generally the same as the one used to equilibrate the column. The pH of the buffer is again maintained between 7 and 9, preferably at pH 8. If sodium sulfate is used as the eluting salt gradient, for example, Tris sulfate will be used as the buffer. The salt gradient ranges between 0 mM to 200 mM.

The protein is collected in fractions of equal aliquots as it comes off the column and is monitored for protein concentration at 280 nanometers in a spectrophotometer. Optionally, in the event that Tris buffer is used, a desalting step using a sizing column is required if the material is to be used for therapeutic purposes. G-10, 15 or 25 Sephadex are appropriate size exclusion resins.

It will be appreciated that the invention includes the use of a hydrophobic interaction or filtration matrix in the purification of a biologically active protein, preferably a tumor necrosis factor protein.

The proteins which may be recovered by the method according to the invention include soluble recombinant ricin toxin A chain.

The invention will be more clearly understood in relation to the following examples which are intended to be merely exemplary and non-limiting.

EXAMPLE I

A stock culture of E. coli K12 strain DG95λ was transformed with pAW711, a plasmid containing cDNA sequences encoding mature humman TNF under the control of $P_L$ promoter. The transformant was deposited with the American Type Culture Collection (ATCC), Rockville, MD USA under No. 39,918 on November 8, 1984. The cells were grown and induced for TNF production before harvesting by centrifugation at 27,000 x g for five minutes.

The cells were washed with 10 mM Tris buffer. pH 7.0, and disrupted by sonication. The cell debris was removed by centrifugation at 27,000 x g for 15 minutes, and the pH of the culture supernatant was adjusted to 8.2 and the salt concentration to 2 mM NaCl.

The supernatant was then applied to the DEAE agarose column, which was equilibrated with 20 mM Tris pH 8.2/1 mM NaCl. The column was then eluted with a gradient of 0-1 M NaCl and 10 mM Tris, pH 8.2. Fractions were collected and assayed for TNF bioactivity, and for protein content by the method of Lowry.

Figure 1 shows the elution profile from the DEAE sepharose column. It is apparent that a portion of the bacterial proteins are not retained by the column and that the TNF activity elutes at a salt concentration level of about 55 mM NaCl.

The fractions from the DEAE column containing TNF activity were pooled and adjusted to 1.8 M ammonium sulfate by the addition of the solid salt and applied to a preparative phenyl-TSK HPLC column previously equilibrated in 1.8 M ammonium sulfate/0.1 M sodium phosphate buffer, pH 7.0. The column was then eluted with a linear gradient of decreasing ammonium sulfate concentration in 0.1 M phosphate buffer pH 7.

Figure 2 shows the elution profile and the successful separation from a contaminating protein of the TNF-containing fractions. The fractions containing TNF activity were then pooled and chromatographed through GH-25 desalting column.

Table 2 below shows the progress of purification in the foregoing procedure. Both the DEAE and the phenyl-TSK HPLC columns effect an approximately tenfold increase in specific activity. Total recovery was 30% after the gel filtration step.

Table 2

Purification of rTNF

| Purification Step | Total Protein (mg) | Units | Specific Activity (U/mg) | % Purity | % Recovery |
|---|---|---|---|---|---|
| Sonicate supernatant | 60 | $200 \times 10^5$ | $3 \times 10^5$ | 5-8 | 100 |
| DEAE Tris-Acryl (ion exchange) | 2 | $100 \times 10^5$ | $5 \times 10^6$ | 60-80 | 50 |
| Phenyl TSK-HPLC (hydrophobic) | 1.6 | $200 \times 10^5$ | $1.2 \times 10^7$ | >95 | 50 |
| GH-25 desalt | 0.6 | $60 \times 10^5$ | $1 \times 10^7$ | >95 | 30 |

In a similar manner, E. coli transformed with plasmids encoding various deletion muteins of TNF were cultured and TNF muteins were extracted. These extracts were subjected to the foregoing procedure to yield homogeneous proteins of comparable purity.

However, the muteins having N-terminal deletions were eluted at different stages of the decreasing salt gradient. While the mTNF illustrated was eluted at 0.4 M ammonium sulfate, ∇7TNF, for example, did not elute for the most part until the ammonium sulfate concentration was reduced to zero and the sodium phosphate to 0.02 M (see Table 1, supra).

The purified proteins were subjected to isoelectric focusing. Lane 1 contained molecular weight markers, lane 2 contained mature recombinant TNF; lanes 3-5 contained muteins of TNF which are missing 4 N-terminal, 7 N-terminal, and 6 N-terminal amino acids, respectively. While all proteins were of similar molecular weight in each case, the mature TNF showed a family of proteins of varying pI values, indicating that possible side chain modification had occurred. This effect was minimized in the muteins.

The purification procedure as set forth in the example for mTNF can be summarized by the flow chart below:

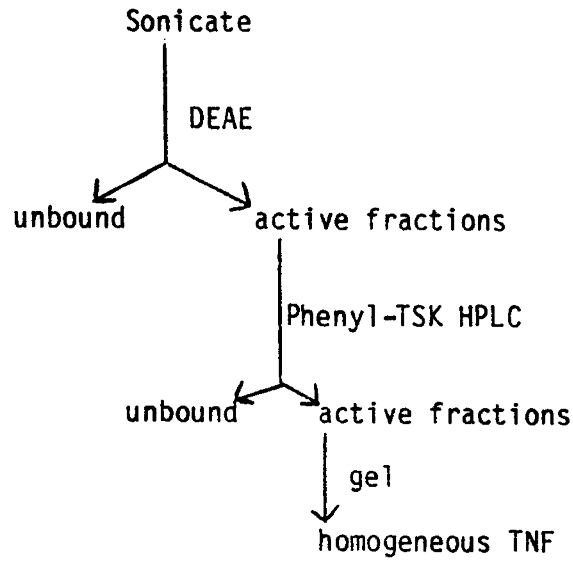


EXAMPLE II

Growth of Recombinant TNF-Producing Host Cells

A. A fermenter was filled with distilled deionized water to operating volume and the following materials were added to the indicated final concentrations: $ZnSO_4.7H_20$, 60 μM; $MnSO_4{\bullet}H_2O$, 60 μM; $CuSO_4{\bullet}5H_2O$, 2 μM; $Na_3citrate{\bullet}2H_2O$, 1.5 mM; $KH_2PO_4$, 21.6 mM; $(NH_4)_2SO_4$, 72 mM. The medium was sterilized in the fermenter. The pH of the medium was adjusted to 6.5± 0.1 with KOH. 50% glucose, KOH and antifoam were added by sterile feeds to the fermenter to achieve a 5 g/l glucose concentration. The following solutions were also added to the indicated final concentrations: 100 μM $FeSO_4.7H_2O$, 20 mg/1 thiamine HCl; 3 mM $MgSO_4.7H_2O$.

B. Inoculum: A stock culture of E. coli K12 strain DG95λ transformed with plasmid pAW740A, the transformant having ATCC Accession No. 53,332 (available from the ATCC, Rockville, MD) was thawed and grown at 30°C to an optical density of 50-100 Klett units in flasks using double strength Luria broth with 10% NaCl, 5 mg/100 ml ampicillin and frozen in vials. One master stock vial was grown in Luria broth as above, but without ampicillin. Culture was diluted to 10% in glycerol and dispensed into vials and frozen at -70°C to be used as working stock.

A container of the working stock was thawed, grown in 2 x Luria broth at 30°C to approximately 1 $OD_{680}$, then added to the fermenter to a final cell concentration of 1 mg/liter.

C. Growth Conditions: Temperature was maintained at 30°C± 1°C, dissolved $O_2$ concentration was 40% air saturation and pH was controlled at 6.8 by automatic addition of 5 N KOH. Optical density was monitored. When the culture reached an $OD_{680}$ of 15 units, temperature was raised to 42°C to induce TNF production and casamino acids were added to 2%. Cells were harvested about four hours after casamino acid addition.

Concentration and Diafiltration

The harvested material was concentrated approximately 5-fold by circulating it under pressure past a hollow fiber microporous (0.2 μ) polypropylene membrane. Residual medium was removed by diafiltration against 5 volumes of deionized water. The retentate was kept and the pH adjusted to 8.2.

Cell Disruption

The concentrated cell suspension was disrupted by multiple passages through a high pressure homogenizer at 6000 to 8000 psig. After disruption the system was washed with deionized water. The disruptate and rinse water were retained and the pH adjusted to 8.2.

Diafiltration on Hydrophobic Membrane at pH 5.5 (Alternative A)

The cell disruptate and rinse water were pH adjusted to 5.5 with glacial acetic acid. The pH adjusted material was diafiltered against 5 volumes of 10 mM acetate buffer using a Dorr-Oliver diafiltration device and a polytetrafluroethylene membrane having a 1.0 μ pore size. The filtrate was collected.

Diafiltration on Hydrophobic Membrane at pH 8.5 (Alternative B)

The cell disruptate and rinse water were treated as indicated above in Alternative A except that a pH of 8.5 was maintained using 10 mM Tris buffer.

Removal of Disrupted Cell Debris:Centrifugation (Alternative 1)

The cell disruptate and rinse water of Alternative A (pH 5.5 diafiltration) were pH adjusted using Tris•HCl and NaOH to 8.2 as necessary and the flocculating agent $CaCl_2$ was added to aggregate cell debris. The aggregate was separated from the supernatant by centrifugation at 14.000 xg in a centrifuge. The supernatant was retained and treated as Diafiltration Alternatives A and B above.

Removal of Disrupted Cell Debris:Diafiltration (Alternative 2)

The cell disruptate and rinse water of Alternative A (pH 5.5 diafiltration) were pH adjusted using Tris•HCl and NaOH to 8.2. The material was diafiltered under pressure at 15 psi against 5 volumes of distilled water using a crossflow hydrophilic cellulose ester hollow fiber cartridge. The filtrate was retained and the pH was adjusted and treated as in Diafiltration Alternatives A and B above.

EXAMPLE III

Concentrating the Filtrate

A. Anion Exchange Chromatography

Tris•HCl was added to the filtrate of Diafiltration (Alternative A) to a concentration of 10 mM and the pH adjusted if necessary to 8.2 with glacial acetic acid or NaOH. An anion exchange column containing quaternary ammonium ion was equilibrated with 10 mM Tris•HCl and the filtrate was loaded onto the cartridge. The loaded material was washed with 65 mM NaCl, 10 mM Tris•HCl pH 8.0. A low molecular weight fraction absorbing at 260 mM eluted with the 65 mM salt wash. A linear 65-300 mM NaCl gradient in 10 mM Tris•HCl pH 8.0 maintained by a gradient controller is used to elute the TNF while monitoring the eluate for protein by absorbance at 280 nM. The protein fraction elutes from the column to yield a TNF-containing eluate having a volume approximately one-twentieth of the filtrate loading volume, and substantially free of nucleic acid degradation products absorbing at 260 nM.

B. Ultra Filtration

The TNF-containing filtrate of Diafiltration (Alternative A) was concentrated 10 to 20-fold by ultrafiltration on a 10.000 molecular weight cut off polysulfone membrane. The TNF remained in the retentate and the retentate was subsequently treated as in Example IV.

EXAMPLE IV

Phenyl Sepharose Chromatography of Filtrate

$(NH_4)_2SO_4$ was added to the filtrate containing TNF obtained in Diafiltration (Alternative A) to a concentration of 1.8 M and the pH was measured and adjusted to 7.0. The material was filtered through a 0.45 micron filter. The filtrate was loaded onto a phenyl Sepharose CL4B column after first equilibrating the column with 1.8 M $(NH_4)_2SO_4$ in sodium phosphate buffer at pH 7.0. Using a gradient controller, a linear gradient of 100% 1.8 M $(NH_4)_2SO_4$, in 10 mM sodium phosphate buffer, at pH 7.0 to 100% of 60% ethylene glycol in 4 mM sodium phosphate buffer pH 7.0, was used to elute the protein from the column. The eluate fractions were monitored for protein concentration at 280 nM and those fractions of TNF falling within 80% of the maximum peak height on the ascending and descending legs of the plot of protein concentration of the eluate fractions were retained and pooled.

EXAMPLE V

$(NH_4)_2SO_4$ was added to the TNF-containing eluate of Example III.A or the retentate of Example III.B to a concentration of 1.8 mM, adjusted to pH 7.0 with HCl, and filtered through a 0.45 µM filter. The sample was subsequently chromatographed on phenyl Sepharose CL4B as in Example IV.

EXAMPLE VI

The pooled eluate from the phenyl Sepharose column is desalted by chromatography on a G-25 Sephadex column equilibrated with 10 mM Tris•HCl buffer pH 9.2. The fast eluting fraction detected by absorbance at 280 nM is collected and subsequently treated as in Example VI, VII, or VIII, except that the dilution with deionized water is omitted unless necessary to achieve a conductivity of less than 2.2 mS.

EXAMPLE VI

The pooled eluate fractions obtained from the phenyl Sepharose column of Example V and optionally desalted were diluted with deionized water to a conductivity less than 2.2 mS and the pH was adjusted to 8.2 with NaOH. A QAE Sepharose column was equilibrated with 10 mM sodium phosphate buffer at pH 8.2 prior to loading the diluted pooled fractions on the column. A linear gradient of 10 mM to 200 mM sodium phosphate buffer pH 8.2 was used to elute the TNF protein from the column. The eluate is monitored at 280 nM for protein concentration of the eluate fractions. The TNF peak falling within 90% of the maximum height on the ascending and descending legs of a plot of eluate fraction protein concentration is retained.

EXAMPLE VII

The pooled eluate fractions from the phenyl Sepharose column were diluted as in Example VI except that 10 mM Tris at pH 8.0 was used. The QAE Sepharose column was equilibrated with 10 mM Tris•HCl buffer at pH 8.0 prior to loading the diluted pooled fractions on the column. A linear 10 to 200 mM NaCl gradient in 10 mM Tris•HCl pH 8.0 was used to elute the TNF from the column. The eluate was monitored and fractions pooled as in Example VI.

EXAMPLE VIII

The pooled fractions from the phenyl Sepharose column were handled as in Example VII except that 10 mM Tris•$SO_4$ pH 8.0 was used to dilute the sample and equilibrate the column. A 10 to 200 mM linear sodium sulfate gradient in 10 mM Tris•sulfate pH 8.0 was used to elute the TNF. Protein concentration was monitored and the TNF fractions were pooled as in Example VI.

EXAMPLE IX

Pooled eluates of Examples VII and VIII are desalted using a G25 Sephadex column equilibrated with 10 mM $NaPO_4$ at pH 8.0. The fast eluting fraction was monitored for protein concentration at 280 nM, pooled and retained.

EXAMPLE X

Determination of TNF Potency-Assay of Biological Activity

TNF activity is quantitatively measured using an in vitro cell cytoxicity assay utilizing a TNF sensitive murine L-929 fibroblast target cell line. Murine L-929 fibroblast cells (ATCC CCL 1.2) are grown in Eagle's Minimum Essential Medium (MEM) with Earle's salts, 10% fetal bovine serum (FBS) and 1% penicillin-streptomycin-fungizone solution, at 37°C (5% $CO_2$). A master stock of the cell culture is stored in liquid nitrogen. Working stocks are subcultured twice weekly and maintained for 30 passages. 96-well flat-bottomed trays containing confluent monolayers of the L-929 cells are prepared by adding 75 µl of 2.6 x $10^5$ trypsinized cells/ml to each well and then incubating the plates for 18 hours at 37° C (5% $CO_2$). Cell monolayers are prepared one day prior to sample addition.

Samples are serially diluted in a separate dilution plate and transferred to the cell plates to which actinomycin-D has been added to a final concentration of 1 ug/ml immediately prior to sample transfer. Cytotoxicity is scored the following day by spectrophotometrically measuring neutral red dye uptake by viable cells. One unit of TNF activity is defined as that amount required for 50% cell killing. This actinomycin-D enhanced cytotoxicity assay is adapted from those described by J. M. Ostrove and G. E. Gifford in Proc. Soc. Exp. Biol. Med., 160:354-358 (1979) and M. R. Ruff and G. E. Gifford in Inf. Imm., 31:380-385 (1981). Scoring cytotoxicity using neutral red staining is modified from the procedure described by F. C. Kull and P. Cuatrecasas in J. Immunol., 126:1279-1283 (1981).

TNF activity of the sample is determined in comparison to a TNF standard prepared from the final purification product of Examples VI, VII, VIII or IX. To prepare the TNF standard, TNF final purification product is diluted with MEM Earl's salts medium containing 2% FBS and 1% penicillin-streptomycin-fungizone solution. The diluted material is aliquoted and stored at -70°C. The L-929 cytotoxicity assay, run on at least six different days, is used to titer the standard, setting 1 unit TNF/ml as the amount of TNF producing 50% cell killing.

As a control, a preparation of TNF is aliquoted into vials, and stored at -70° C. In each assay, a vial is assayed along with the other samples for the purpose of evaluating inter-assay variability.

The quantitative measurement of the TNF activity of a sample is performed as follows:

The sample is diluted in assay medium (MEM Earl's salts medium containing 2% FBS and 1% penicillin-streptomycin-fungizone solution) to estimated TNF concentrations between $10^4$ and $10^5$ units/ml.

An aliquot of the TNF control sample is diluted in assay medium to estimated TNF concentrations between $10^4$ and $10^5$ units/ml.

96-well trays are filled with 120µl/well assay medium. 60 µl of one of the following TNF solutions is added to the first well of each row and is serially diluted 1:3 down each row:

a. The sample to be assayed.
b. TNF control sample.
c. In-house TNF standard.

The sample plates are UV sterilized for 10 minutes and then incubated for approximately 10 minutes at 37° C (5% $CO_2$).

25 µl/well of actinomycin-D (1 µg/ml final concentration) is added to 96-well flat-bottomed trays containing confluent monolayers of L-929 cells (74 µl), and within two hours 100 µl/well of the serially diluted samples is added. Assay plates are incubated for 18 hours at 37° C (5% $CO_2$) and the cells are stained with 50 µl/well of neutral red (0.075%)/glutaraldehyde (10.17%) solution. Plates are incubated for one hour at 37° C (5% $CO_2$) and excess staining solution is removed. Plates are washed with 600 µl/well of PBS. 100 µl/well of stain solubilizing solution (3% SDS, 0.04 N HCl in 2-propanol) is added and the plates are shaken for one minute. Absorbance is read at 550 nm using a plate reader.

From the dilution factor required to each 50% cell killing, plate-to-plate variation is corrected by means of the in-house TNF standard. The cytotoxicity activity in the pre-diluted samples is calculated and expressed as

units/ml. If the sample was diluted the activity is multiplied by the pre-dilution factor to obtain the units/ml in the sample.

TNF concentration and specific activity can be determined with this information and the protein concentration obtained by standard Lowry test.

EXAMPLE XI

Protein Purity Assay:SDS-PAGE

To determine the purity of the TNF protein, the final purification product is analyzed by SDS - polyacrylamide gel electrophoresis (SDS-PAGE), which separates proteins by molecular sizing. Quantitative measurement of protein impurities is obtained from Fast Green-stained gels. The qualitative but highly sensitive evaluation of impurities is obtained from silver-stained gels. The quantitative measurement of protein impurities in the final purification product is performed on both reducing and non-reducing gels using Fast Green-staining as follows:

For reducing gels, TNF final purification product is diluted in buffered 2-mercaptoethanol solution (10% mercaptoethanol, 18% glycerol, 4% SDS and 0.11 M Tris pH 6.8) to a concentration of about 0.25 mg/ml. This protein solution is heated for three minutes at approximately 95°C. Calibration proteins are treated in an identical manner.

For non-reducing gels, TNF final purification product is diluted in buffered solution (20% glycerol, 4% SDS and 0.12 M Tris pH 6.8) to a concentration of about 0.25 mg/ml and this protein solution is heated for three minutes at approximately 95°C.

The SDS-PAGE apparatus is set up containing a 1.5 mm thick gel of a linear 10-15% acrylamide gradient. Approximately 7 µg protein/gel lane (at least 2 lanes per sample), is loaded and electrophoresed. After electrophoresis, the gel slab is stirred in a solution containing 1% Fast Green and 7% acetic acid. The gel is destained with a solution containing 5% methanol and 7% acetic acid and scanned on a densitometer at wavelength 635 nm.

The area of the main peak (assignable to TNF) is measured, divided by the total area measured in the scan (areas due to inclusions in the gel or scratches on the gel carrier plate are subtracted from the total area measurement) and multipled by 100, to determine the percent of the staining area that is attributable to TNF.

The qualitative evaluation of impurities in the final purification product is performed on both reducing and non-reducing gels using silver-staining. Gels 0.75 mm thick of 15% acrylamide are used. Approximately 1 µg of protein is loaded into a lane. After electrophoresis, silver-staining is accomplished by placing the gel slab in a fixing solution of ethanol/acetic acid, then in a staining solution of silver nitrate/sodium hydroxide-ammonium hydroxide, and finally in a developing solution of formaldehyde/citric acid. The gels are then photographed.

EXAMPLE XII

Protein Purity Assay - Isoelectric Focusing

To determine the purity of the TNF protein, the product also is analyzed by isoelectric focusing (IEF), which separates proteins by their isoelectric point (pI). The qualitative but sensitive evaluation of protein species is obtained from Coomassie Blue-stained gels. The measurement of protein species in the product is performed on polyacrylamide gels as follows:

The IEF flat bed apparatus FBE 3000 is set up with a pH 4.0-6.5 polyacrylmide gel (PAG) plate and a pH 3.5-9.5 PAG plate. Approximately 3 µg protein is loaded per gel lane for each sample and 10 µg/gel lane for the pI standards.

The sample is electrofocused and after electrofocusing, the gels are placed in a fixing solution containing 3.8% 5-sulfosalicylic acid, 12% trichloroacetic acid and 30% methanol. The gels are washed with a solution containing 25% ethanol and 8% acetic acid and stained in a solution containing 0.09% Coomassie Brilliant Blue R, 25% ethanol and 8% acetic acid.

The gels are destained with a solution containing 25% ethanol and 8% acetic acid.

EXAMPLE XIII

The Limulus Amebocyte Lysate (LAL) test, as described in USP XX, page 888, is used to assess the level of endotoxin present in the final purification product. Lyophilized preparations of lysate and control standard endotoxin are obtained from licensed vendors for use in the test. The LAL test on the final purification product is performed as follows:

Final purification product is suspended in Sterile Water for Injection, U.S.P. Four replicate 2-fold dilution series for the suspended product and for the control standard endotoxin using Sterile Water for Injection, U.S.P. as diluent are prepared. Negative controls consisting of the sterile water diluent only and positive controls consisting of the same diluent inoculated with endotoxin at a level of not more than two times the stated lysate sensitivity are included.

Lysate is added to each tube and the tube is incubated at 37 ± 1°C for 60 ± 2 minutes and read. The concentration of endotoxin in the sample is calculated by the formula (p$\lambda$) (f/$\Sigma$) as described in USP XX.

For an acceptable test, the following conditions must be met:

The lysate sensitivity obtained in the test must be within one serial dilution of the labeled sensitivity. The

negative control must exhibit no gelation and firm gelation must occur in the positive control(s).

EXAMPLE XIV

The product of Example IX is diluted in 20 mM sodium phosphate pH 7.5 concentration sufficient to yield a predetermined specific activity when mixed with a solution of 20% mannitol. 20% mannitol is added and the formulated product is prefiltered through a 0.45 µM filter. The formulated product is lyophilized.

EXAMPLE XV

The desalted product of Example X is obtained and formulated as in Example XIV.

The above-described purified TNF may be formulated with any one of a number of well known pharmaceutically acceptable carriers, depending upon the optimal route of administration, e.g., parenteral, including intravenous, intraperitoneal, intramuscular and subcutaneous. Such carriers include solutions compatible with the mode of administration and solubility of the compounds. Such solutions may be buffered or otherwise formulated to minimize undesirable localized effects of injection if necessary.

For parenteral use, the compounds of this invention can be formulated with sterile ingredients compounded and packaged aseptically. They may be administered intravenously or intramuscularly. Useful solvents for formulation in such use are the polyhydric aliphatic alcohols and mixtures thereof. Pharmaceutically acceptable glycols, such as propylene glycol, and mixtures thereof, or glycerine may be employed. Pharmaceutically acceptable sugar alcohols such as mannitol or sorbitol may be used. Water may be incorporated in the vehicle if desired.

A pH range of about 7.4 and isotonicity compatible with body insotonicity are desirable. Basicity may be controlled by the addition of a base as required. It may often be desirable to incorporate a local anesthetic, and such are well known to those skilled in the art. The percentage of the compound to be used in the pharmaceutical carrier may be varied. It is necessary that the compound constitute a proportion such that a suitable dosage will be obtained.

The dosage required to achieve the desired pharmacologic activity in the mammal will vary with various factors such as route of administration, the species of mammal, general health and tolerances of the mammal, weight, sex and age of the mammal, the nature and severity of the disease being treated, and the like. Additionally, it is to be noted that the exact dosage of each individual compound employed in similar situations will vary.

The invention further relates to a pharmaceutical or veterinary formulation comprising a novel TNF as hereinbefore described formulated for pharmaceutical or veterinary use respectively, optionally comprising a pharmaceutically or veterinarily acceptable carrier, diluent or excipient, and/or optionally in unit dosage form. The invention further relates to a method of making a pharmaceutical or veterinary formulation which comprises formulating a novel TNF as hereinbefore described, optionally together with a pharmaceutically or veterinarily acceptable carrier, diluent or excipient, and/or optionally into unit dosage form.

The invention further relates to the use of a TNF as hereinbefore described in the preparation of an anti-tumour medicament.

**Claims**

1. A method for partially purifying a biologically active protein wherein such protein is produced in a recombinant host comprising the steps of passing a fluid containing the biologically active protein through a continuous hydrophobic porous matrix and recovering the partially purified biologically active protein.

2. A method as claimed in claim 1 wherein the protein is recovered in the filtrate or retentate and is recombinant tumor necrosis factor or soluble recombinant ricin toxin A chain.

3. A method for purifying tumor necrosis factor (TNF) comprising by the steps of:

(a) treating an aqueous non-silica interaction mixture containing TNF with a hydrophobic matrix under a first salt concentration condition effective to result in retention of TNF on the hydrophobic matrix, but ineffective to precipitate the TNF, and

(b) eluting the TNF from the hydrophobic matrix chromatographically by decreasing the salt concentration below the first salt concentration of step (a).

4. A method as claimed in claim 3 wherein the TNF is recombinant TNF, the mixture contains bacterial proteins and is free of other mammalian proteins, the steps are carried out using high pressure liquid chromatography, first salt concentration conditions comprise 1.5-2.0 M ammonium sulfate and 0.1 M sodium phosphate, pH7, and the decrease in salt concentration is by continuous gradient.

5. A method as claimed in claim 3 or claim 4 wherein before step (a), and without a desalting step, the TNF is eluted from an anion exchange support matrix in a salt concentration high enough to elute an aqueous mixture containing TNF from the column but not precipitating TNF from aqueous solution.

6. A purified TNF composition prepared by the method of any one of claims 3-5, said TNF having an amino acid sequence substantially equivalent to mature TNF and modifications of the primary amino acid sequence of mature TNF that do not completely destroy the biological activity thereof in in

vitrocytotoxicity assay on L-929.

7. A TNF composition as claimed in claim 6 wherein the modifications, simple or in combination are N-terminal deletions, cysteine-depleted muteins. amino acids additions, and/or amino acid substitutions.

8. A method for partially purifying tumor necrosis factor (TNF) from a TNF-containing fluid obtained from TNF-producing recombinant host cells comprising the steps of:

(a) passing the TNF-containing fluid through a continuous hydrophobic porous matrix, and

(b) recovering a partially purified TNF in which the TNF comprises in the range of 40 to 50% by weight of the total protein, the TNF comprises at least about 20% by weight of the total TNF produced by the recombinant host cells, and the TNF has an endotoxin content in the range of 10 ng/ml to 10 μg/ml.

9. A method for purifying tumor necrosis factor (TNF) from a TNF-containing fluid obtained from TNF-producing recombinant host cells comprising steps of:

(a) passing the TNF-containing fluid through a continuous hydrophobic porous matrix to produce a partially purified TNF:

(b) further purifying said partially purified TNF by at least one hydrophobic interaction matrix chromatography step, and at least one anion exchange matrix chromatography step, and

(c) recovering a purified TNF having a TNF content of at least 95% as determined by SDS-PAGE analysis and an endotoxin content of less than 0.1 ng/mg TNF.

10. A method as claimed in claim 9 wherein at least one anion exchange chromatography step precedes at least one hydrophobic interaction matrix chromatography step, optionally further including the steps of desalting the TNF-containing eluate from the hydrophobic interaction matrix and chromatographing the desalted TNF-containing eluate on an anion exchange column.

11. A method as claimed in claim 9 wherein at least one chromatography step on a hydrophobic interaction matrix precedes said anion exchange step.

12. A method for purifying tumor necrosis factor (TNF) from TNF-producing recombinant host cells comprising the steps of:

(a) disrupting the host cells at a pH range between 7.5 and 9.5;

(b) removing cell debris at pH between 7.5 and 9.5 to produce a TNF-containing fluid;

(c) passing the TNF-containing fluid through a porous polytetrafluoroethylene (PTFE) membrane at a pH in a range of 5.0 and 9.5 to form a partially purified TNF; and

(d) further purifying the partially purified TNF by elution from a hydrophobic hydrocarbyl agarose and elution from an anion exchange matrix and recovering a purified TNF having a TNF content of at least 95% as determined by SDS-PAGE analysis and an endotoxin content of less than 0.1 ng/mg TNF.

13. A method as claimed in claim 12 wherein in step (d) sequential elutions occur with at least one desalting after the elution from said hydrophobic hydrocarbyl agarose, in that said PTFE membrane has pores in a range between 0.1 ad 3.0 microns, and said elution from the anion exchange matrix comprises eluting the TNF with a high salt concentration.

14. A purified recombinant tumor necrosis factor (TNF) composition having a TNF content of at least about 95% as determined by SDS-PAGE analysis, and an endotoxin content of less than about 0.1 ng/mg, said TNF being substantially free of pyrogens as determined by the USP rabbit pyrogen test at a dosage range of $1.0 \times 10^5$ to $2.4 \times 10^5$ U/kg.

15. A purified TNF composition as claimed in claim 14 wherein the TNF is N-terminally deleted at one of N-terminal positions 1 to 10.

16. A purified TNF composition as claimed in claim 15 characterized in that the TNF is N-terminally deleted at N-terminal position 8.

17. A pharmaceutical or veterinary formulation comprising a TNF composition as claimed in any one of claims 6 or 7 or 14 to 16 formulated for pharmaceutical or veterinary use respectively, optionally comprising an acceptable carrier, diluent or excipient and/or optionally in unit dosage form.

18. A method for making a formulation as claimed in claim 17 which comprises formulating a TNF composition as claimed in any one of claims 6 or 7 or 14 to 17, optionally together with a pharmaceutically or veterinarily acceptable carrier, diluent or excipient, and/or optionally into unit dosage form.

19. The use of a TNF as claimed in claim 6 or claim 14 in the preparation of an anti-tumor medicament.

20. The use of a hydrophobic interaction or filtration matrix in the purification of a biologically active protein, preferably a tumor necrosis factor protein.

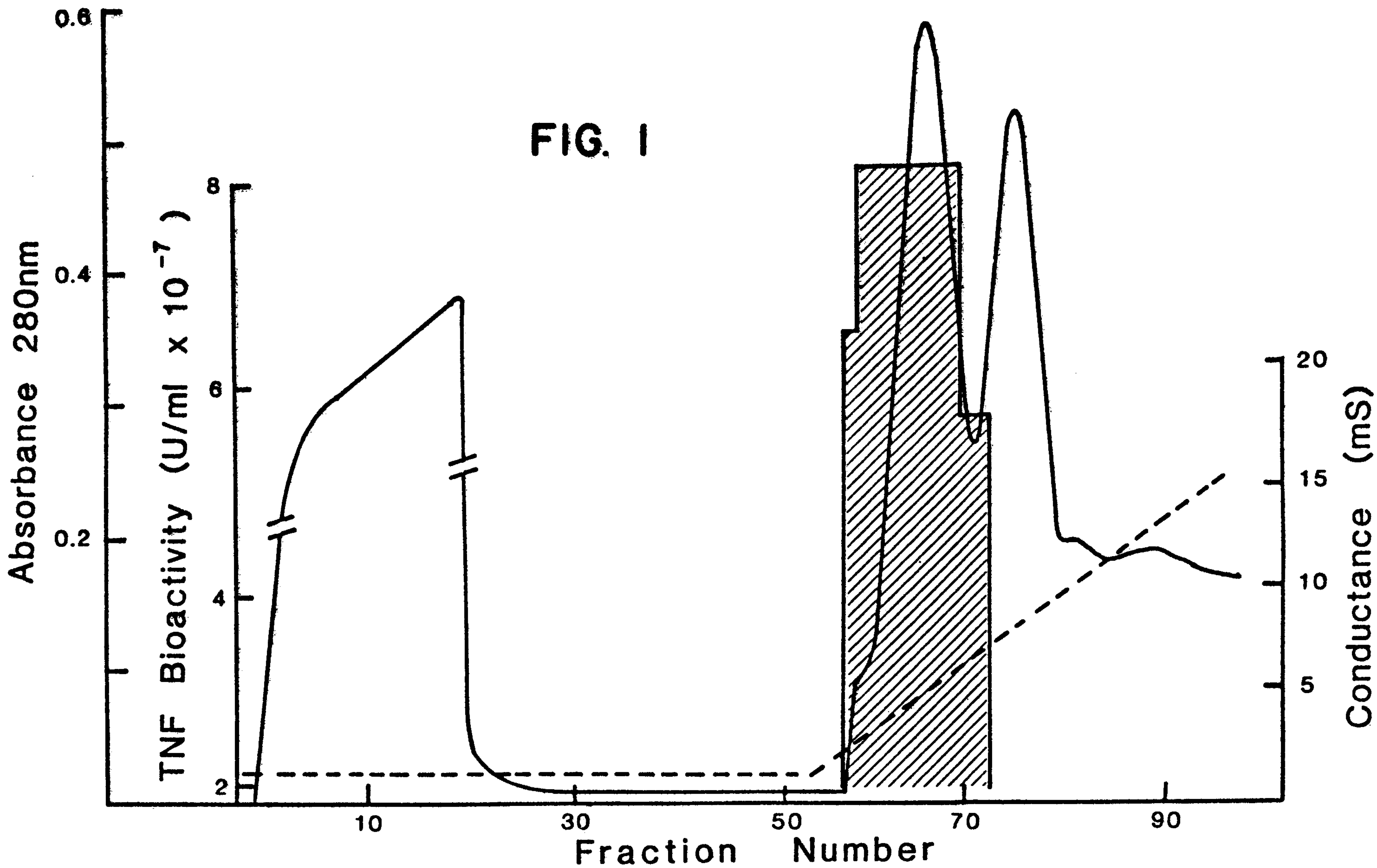


FIG. I

0220966

FIG. 2

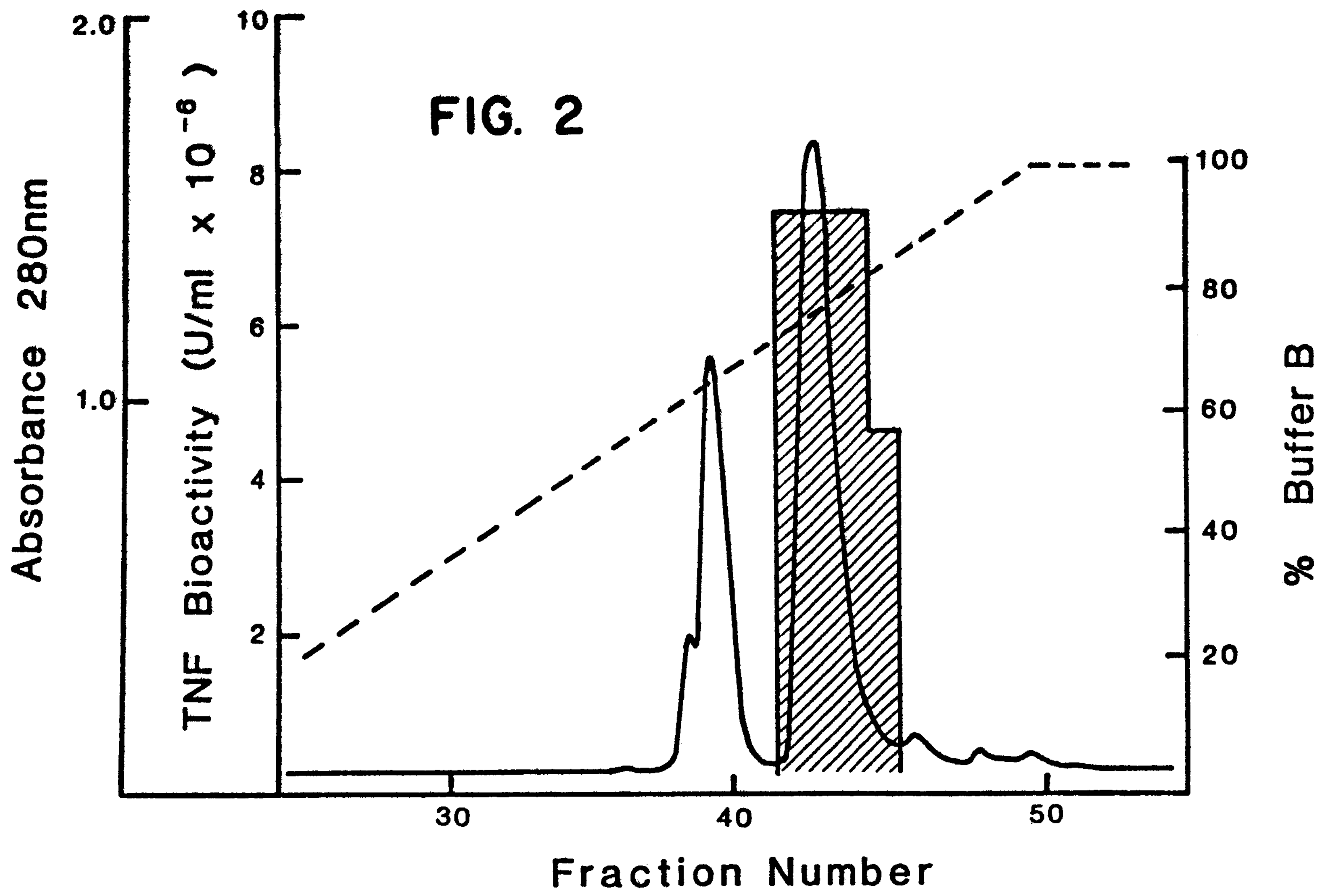
Absorbance 280nm
2.0
1.0
TNF Bioactivity (U/ml x 10$^{-6}$ )
10
8
6
4
2
100
80
60
40
20
% Buffer B
30
40
50
Fraction Number

# FIG. 3

▽1 ▽2 ▽3 ▽4 ▽5 ▽6 ▽7 ▽8 ▽9 ▽10

1 ValArgSerSer SerArgThrPro SerAspLysPro ValAlaHisVal ValAlaAsnPro

▽31

21 GlnAlaGluGly GlnLeuGlnTrp LeuAsnArgArg AlaAsnAlaLeu LeuAlaAsnGly

41 ValGluLeuArg AspAsnGlnLeu ValValProSer GluGlyLeuTyr LeuIleTyrSer

ALA 69
SER 69

61 GlnValLeuPhe LysGlyGlnGly CysProSerThr HisValLeuLeu ThrHisThrIle

81 SerArgIleAla ValSerTyrGln ThrLysValAsn LeuLeuSerAla IleLysSerPro

ALA101
SER101

101 CysGlnArgGlu ThrProGluGly AlaGluAlaLys ProTrpTyrGlu ProIleTyrLeu

121 GlyGlyValPhe GlnLeuGluLys GlyAspArgLeu SerAlaGluIle AsnArgProAsp

▽140 ▽150 ▽156

141 TyrLeuAspPhe AlaGluSerGly GlnValTyrPhe GlyIleIleAla Leu

FIG. 4

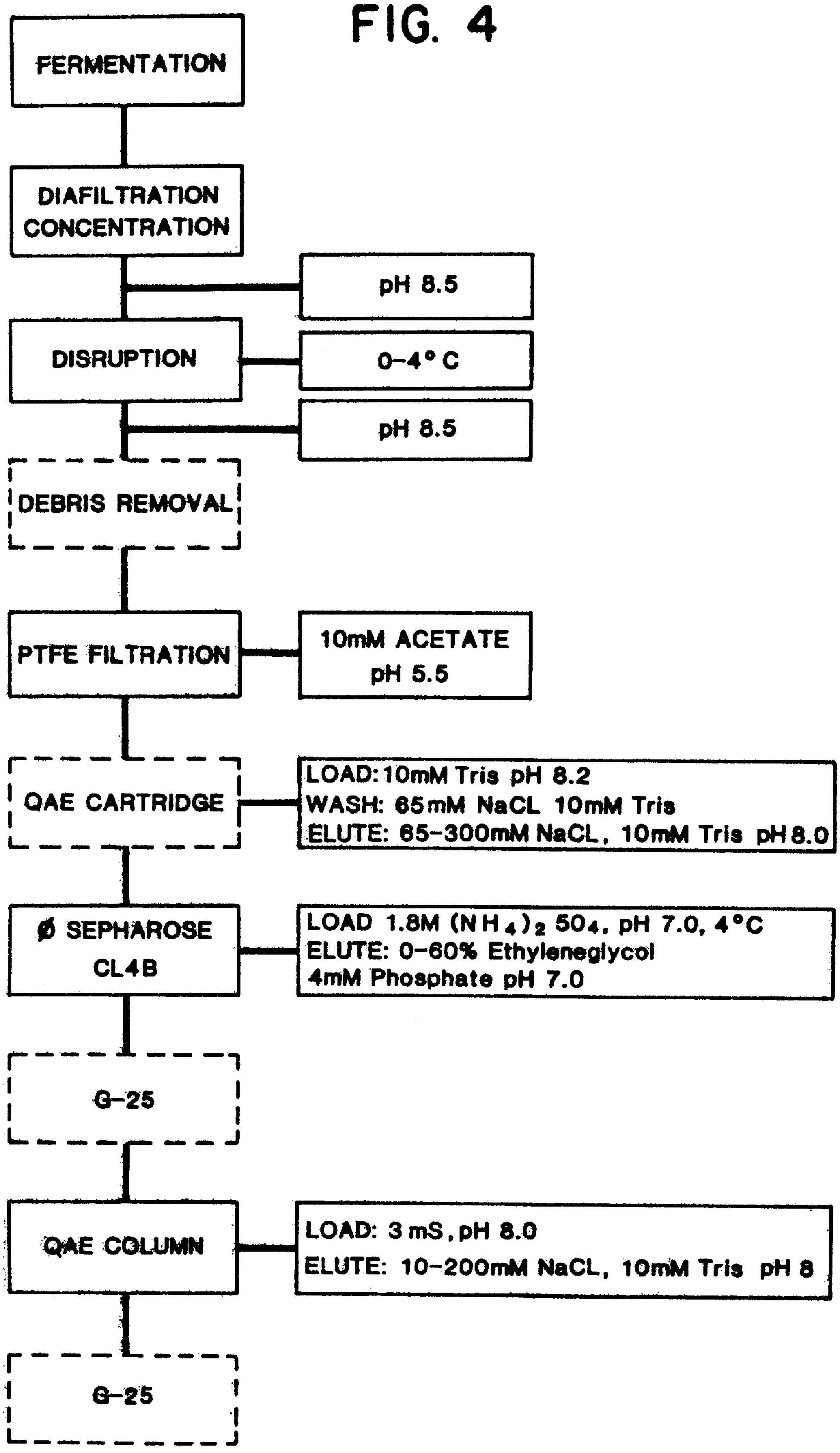
FERMENTATION
DIAFILTRATION CONCENTRATION
pH 8.5
DISRUPTION
0-4° C
pH 8.5
DEBRIS REMOVAL
PTFE FILTRATION
10mM ACETATE pH 5.5
QAE CARTRIDGE
LOAD: 10mM Tris pH 8.2
WASH: 65mM NaCL 10mM Tris
ELUTE: 65-300mM NaCL, 10mM Tris pH 8.0
ø SEPHAROSE CL4B
LOAD 1.8M (NH4)2 SO4, pH 7.0, 4°C
ELUTE: 0-60% Ethyleneglycol
4mM Phosphate pH 7.0
G-25
QAE COLUMN
LOAD: 3 mS, pH 8.0
ELUTE: 10-200mM NaCL, 10mM Tris pH 8
G-25

FIG. 5

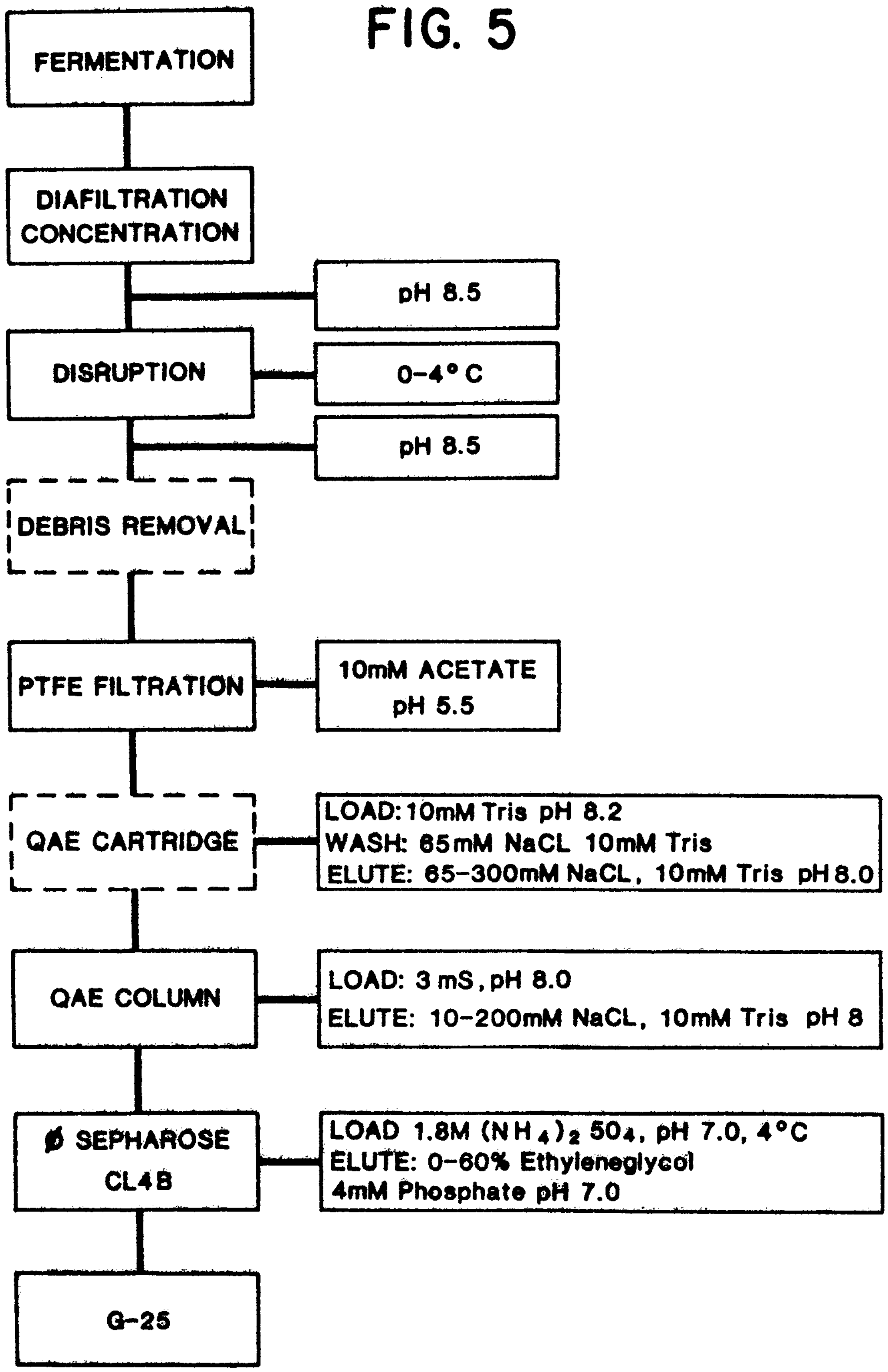
FERMENTATION
DIAFILTRATION CONCENTRATION
pH 8.5
DISRUPTION
0-4° C
pH 8.5
DEBRIS REMOVAL
PTFE FILTRATION
10mM ACETATE pH 5.5
QAE CARTRIDGE
LOAD: 10mM Tris pH 8.2
WASH: 65mM NaCL 10mM Tris
ELUTE: 65-300mM NaCL, 10mM Tris pH 8.0
QAE COLUMN
LOAD: 3 mS, pH 8.0
ELUTE: 10-200mM NaCL, 10mM Tris pH 8
ø SEPHAROSE CL4B
LOAD 1.8M (NH4)2 SO4, pH 7.0, 4°C
ELUTE: 0-60% Ethyleneglycol
4mM Phosphate pH 7.0
G-25